# EUROPEAN PATENT SPECIFICATION

(11) **EP 2 480 231 B1**
(45) Date of publication and mention of the grant of the patent: **10.05.2017**
(21) Application number: 10779347.3
(22) Date of filing: 17.09.2010
(51) Int. Cl.: A61K 31/401, A61K 31/403, A61K 31/551, A61K 31/675, C07D 487/04, C07D 207/16, C07D 209/42, A61K 45/06, A61P 9/00, A61P 9/10, A61P 9/12

(54) **NEW SALTS OF PERINDOPRIL WITH BENZATHINE, PROCESS FOR THEIR PREPARATION AND THEIR USE FOR THE TREATMENT OF CARDIOVASCULAR DISEASES**
NEUE SALZE VON PERINDOPRIL MIT BENZATHIN, VERFAHREN ZU DEREN HERSTELLUNG UND DEREN VERWENDUNG BEI DER BEHANDLUNG KARDIOVASKULÄRER ERKRANKUNGEN
NOUVEAUX SELS DE PERINDOPRIL AVEC LA BENZATHINE, PROCÉDÉ POUR LEUR PRÉPARATION ET LEUR UTILISATION POUR LE TRAITEMENT DE MALADIES CARDIOVASCULAIRES

(30) Priority: 21.09.2009 SI 200900255
(43) Date of publication of application: 01.08.2012
(73) Proprietor: Silverstone Pharma, 9490 Vaduz (LI)
(72) Inventor: Rucman, Rudolf, 1211 Ljubljana (SI); Zupet, Pavel, 8000 Novo mesto (SI)
(74) Representative: Ros, Zlata
(86) International application number: PCT/SI2010/000052
(87) International publication number: WO 2011/034509

(56) References cited:
- EP-A1- 0 391 462
- EP-A1- 0 545 194
- EP-A1- 1 296 947
- WO-A1-99/11260
- WO-A1-2004/041290
- WO-A1-2008/065431
- WO-A2-01/80847
- JP-A- 2004 250 447
- SUH J T ET AL: "Angiotensin-converting enzyme inhibitors. New orally active antihypertensive (mercaptoalkanoyl)- and [(acylthio)alkanoyl]glycine derivatives.", JOURNAL OF MEDICINAL CHEMISTRY JAN 1985 LNKD- PUBMED:2981324, vol. 28, no. 1, January 1985 (1985-01), pages 57-66, XP7917592, ISSN: 0022-2623
- SCHWAB A ET AL: "Inhibition of angiotensin-converting enzym by derivaties of 3-mercapto-2-methyloropanoyl glycine", BIOCHEMICAL PHARMACOLOGY, PERGAMON, OXFORD, GB, vol. 32, no. 12, 15 June 1983 (1983-06-15) , pages 1957-1960, XP023728404, ISSN: 0006-2952, DOI: DOI:10.1016/0006-2952(83)90067-9 [retrieved on 1983-06-15]
- BYRN S R ET AL: "Chemical reactivity in solid-state pharmaceuticals: formulation implications.", ADVANCED DRUG DELIVERY REVIEWS 16 MAY 2001 LNKD- PUBMED:11325479, vol. 48, no. 1, 16 May 2001 (2001-05-16), pages 115-136, XP002628347, ISSN: 0169-409X
- BASTIN R J ET AL: "Salt Selection and Optimisation for Pharmaceutical New Chemical Entities", ORGANIC PROCESS RESEARCH AND DEVELOPMENT, CAMBRIDGE, GB, vol. 4, no. 5, 1 January 2000 (2000-01-01) , pages 427-435, XP002228592, DOI: DOI:10.1021/OP000018U
- CAIRA M R: "CRYSTALLINE POLYMORPHISM OF ORGANIC COMPOUNDS", TOPICS IN CURRENT CHEMISTRY, SPRINGER, BERLIN, DE, vol. 198, 1 January 1998 (1998-01-01), pages 163-208, XP001156954, ISSN: 0340-1022, DOI: DOI:10.1007/3-540-69178-2_5 ISBN: 978-3-540-36760-4

## Description

### Technical Field

The present invention belongs to the field of pharmaceutical chemistry and relates to new stable salts of perindopril with N,N'-dibenzylethylenediamine in amorphous and crystal forms of formula: 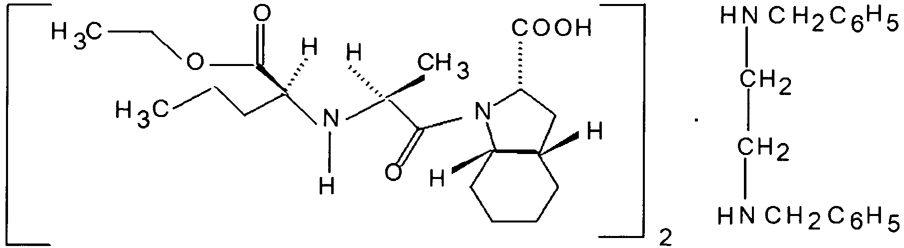 a process for the preparation thereof and the use thereof for the preparation of a pharmaceutical form for the treatment of cardiovascular diseases associated with hypertension.

### Technical Problem

In the practical application of hitherto known salts of ACE inhibitors, one often encounters an insufficient stability of pharmaceutical preparations. A marked example thereof are preparations containing perindopril erbumine and therefore their use has lately been increasingly discontinued.

Thus, there existed a need for new salts with better stability so that the prepared pharmaceutical preparations would also be applicable in countries with higher average day temperatures and an increased air humidity and will have a sufficiently long shelf-life.

### Prior Art

ACE (Angiotensin Converting Enzyme) inhibitors are very useful in the treatment of cardiovascular diseases, especially those associated with hypertension and weakened heart muscle function. There are known numerous ACE inhibitors such as enalapril, quinapril, perindopril, trandolapril, ramipril, fosinopril, spirapril, moexipril, lisinopril, cilazapril and others.

For the production of a pharmaceutical form suitable for application, they can be used in the basic form (free acid), as a hydrate, but mostly in the form of a suitable salt, particularly due to the better solubility in water.

Perindopril is usually a salt with tert-butylamine (erbumine) in various polymorph crystal forms: alpha, beta, gamma, delta, epsilon, eta and D, disclosed in EP-A 0 308 341, EP 1 296 947 B1, EP 1 294 689 A1, EP 1 296 948, WO 2007/02009A1, as erbumine in hydrated form disclosed in EP 1 647 547 and WO 2004/46172 and as a salt with amino acid arginine disclosed in EP 1 354 873.

Perindopril and also other ACE inhibitors are often combined with diuretics e.g. with hydrochlorothiazide, indapamide and others as described in WO 2007/099217A1.

The main problem with the majority of ACE inhibitors is a relatively low stability of the compounds. Several chemical reactions take place, which lead to a decomposition of the molecule, to the formation of new impurities and to a decrease of activity. In the first place is certainly the elimination of water followed by internal cyclization and the formation of diketopiperazine impurities. Diketopiperazine compounds are inactive and decrease the effectiveness of the basic compound.

A second undesired reaction is the hydrolytic cleavage of the ester group under formation of dicarboxylic acid such as perindoprilat. This compound is several times more reactive than the basic compound, yet its transport within the organism is more difficult. The cleavage of the ester bond is stimulated by strong alkaline ions and a higher water content in the preparation and by esterase enzymes within the organism.

The problem of stability is predominantly solved by precisely performed methods of preparation of the pharmaceutical form, granulation (dry or wet), and also by several additives for increased stability. With perindopril erbumine mostly bicarbonates or carbonates of alkali metals or alkaline earth metals are added (US 2003/0215526A1).

Certainly, the stability of a pharmaceutical preparation with ACE inhibitors also strongly depends upon the basic stability of the salt containing ACE inhibitor. In the case of perindopril, it is known that other salts such as calcium, sodium or argininium salt are substantially more stable than erbumine.

### Brief Description of the Figures

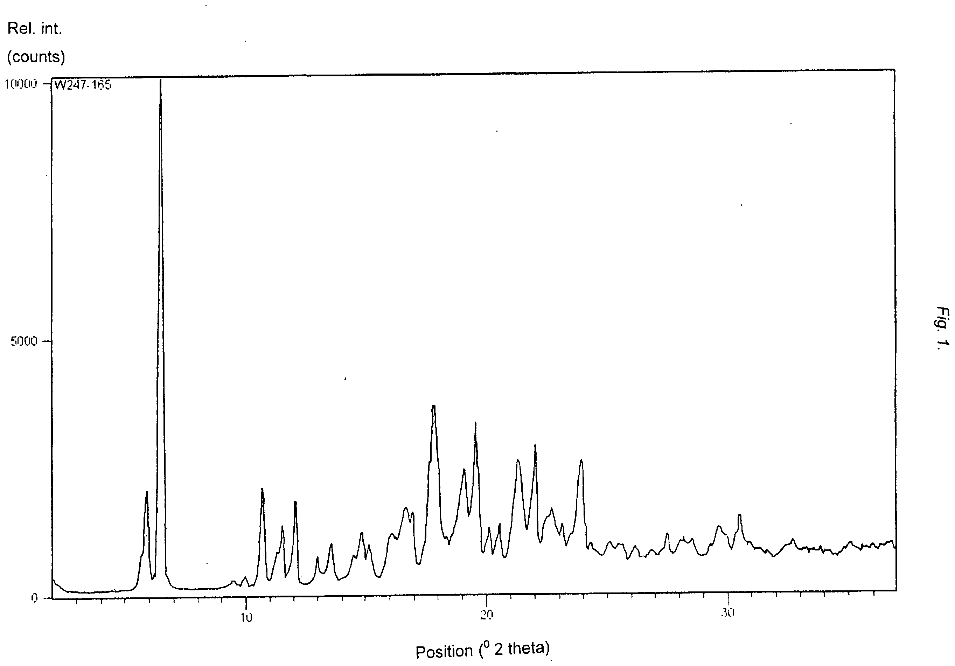

- Fig. 1.: X-ray powder diffractogram of benzathine perindopril, polymorph form A.

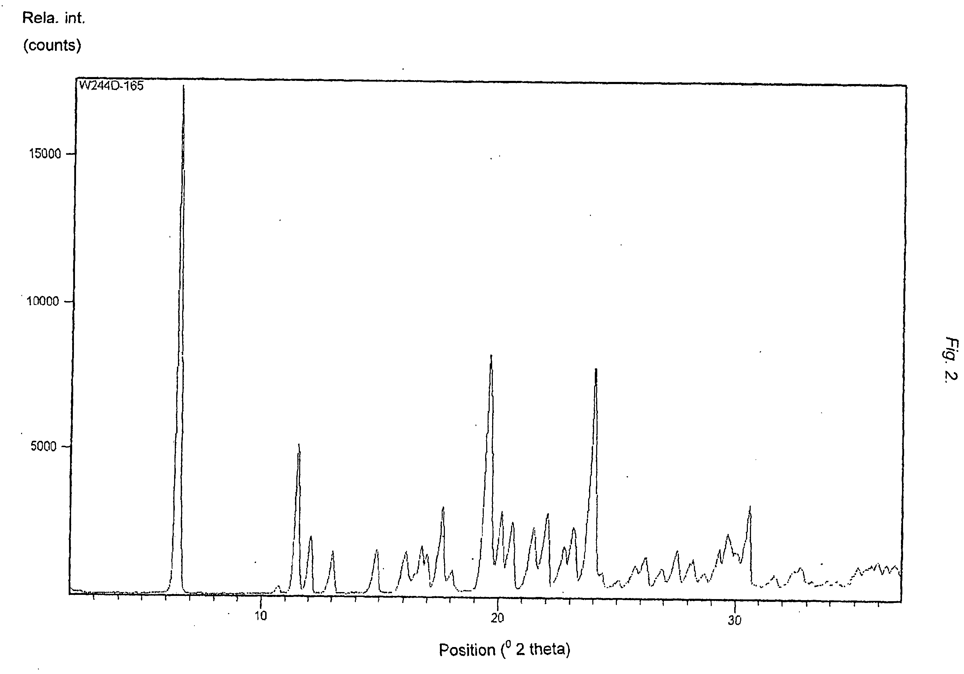

- Fig. 2.: X-ray powder diffractogram of benzathine perindopril, polymorph form B.

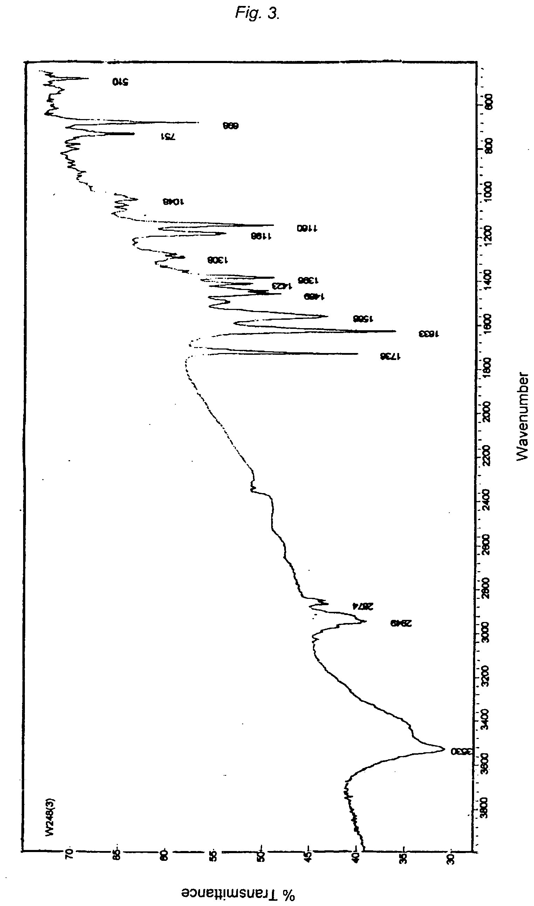

- Fig. 3.: FTIR spectrum of benzathine perindopril, polymorph form A.

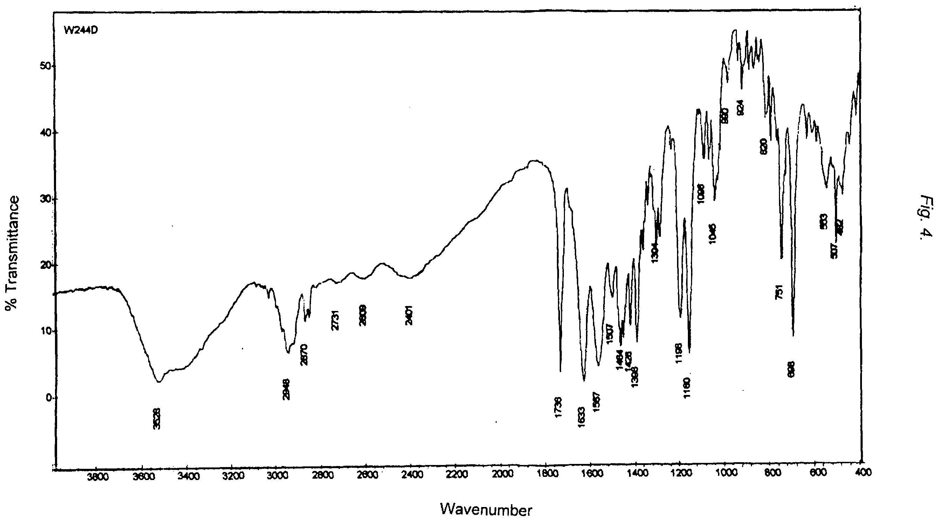

- Fig. 4.: FTIR spectrum of benzathine perindopril, polymorph form B.

### The Technical Solution

The main technical problem associated with the stability of perindopril and salts thereof is solved in several ways. Primarily, it is necessary to prevent the hydrolysis of the ester group and the internal cyclization to diketopiperazine products. This can be affected by the selection of an appropriate cation of the salt, by steric conditions in the salt, by the selection of appropriate inert additives necessary for the preparation of the granulate for tabletting, by various secondary additives for the increase of stability and by the method of the preparation of the pharmaceutical form.

In the case of perindopril erbumine it has turned out that tert-butylamine, which is a component of the addition salt, is linked in a relatively weak manner. After the elimination of tert-butylamine from the salt, a free perindopril acid is formed, which is highly susceptible to nucleophilic attack and internal cyclization. This can be partly solved by the formation of a ionic salt, e.g. with potassium, sodium or calcium cations as described in WO 2008/150245A2.

The conformational analysis of perindopril salt structure by ¹H-NMR spectroscopy has shown that the molecule exists in cis and trans forms. The trans form is more present with perindopril erbumine. However, it is characteristic that just in the cis conformer an intermolecular formation of a hydrogen bond between the carboxylate group and the NH group is present, which substantially stabilizes the molecule! Suprisingly, it has been found that in the salts of perindopril with N,N'-dibenzylethylenediamine (abbr. benzathine) generally a greater part of the cis conformer is present, which is the reason for a greater stability of synthetized benzathine salts of perindopril.

Another possible reason for the low stability of some salts of perindopril is the hydrolysis of the ester bond. It is stimulated by a higher water content, a higher temperature and in the presence of substances with a strong alkaline action such as tert-butylamine or Na ions. Also from this aspect, the selection of N,N'-dibenzylethylenediamine as a component of the salt is very appropriate.

An object of the present invention are the salts of perindopril with N,N'-dibenzylethylenediamine (abbr. benzathine) of formula: 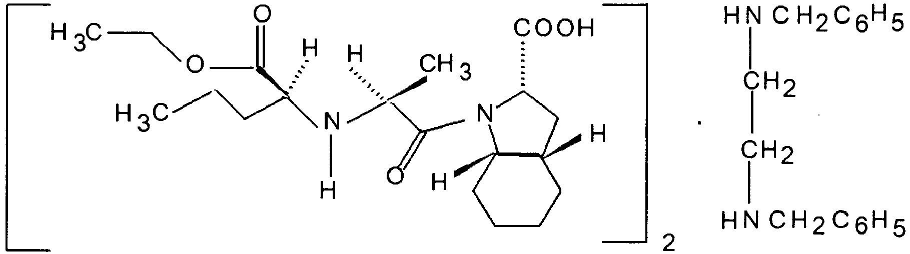 wherein the molar ratio of perindopril to benzathine is 2 : 1.

The advantages of benzathine salts of perindopril are as follows:
- the salts possess a very good stability at ageing since almost no hydrolysis of ester groups and no formation of diketopiperazine impurities occur;
- the salts crystalize excellently from usual solvents and some also from water;
- by crystalization the product purifies itself very well of synthesis admixtures and very pure active substances are obtained;
- crystallized compounds have a higher melting point than amorphous ones and also surpass them in stability;
- benzathine is a nontoxic component and is well known in the field of penicillin antibiotics.

Otherwise, benzathine salt of perindopril can also be prepared in an amorphous form if the salt solutions are frozen and then lyophilized. The yields of the synthesis are higher, yet the salts are slightly less pure.

According to the invention, the benzathine salt of perindopril is prepared by the reaction of both components in a molar ratio of 2 moles of perindopril in the form of a free acid to 1 mole of benzathine and crystallization in appropriate solvents that are defined separately for each case.

As an exception, benzathine perindopril can also be obtained by wet granulation so that onto the inert components - granulating mass ingredients - a solution of perindopril erbumine and benzathine is sprayed and it is dried in a counterflow of warm air. Thereby tert-butylamine evaporates and benzathine perindopril remains included on the granulate. The granulate is then tabletted.

As an other exception, benzathine perindopril can also be obtained in industrial production immediately after step of catalytic debenzylation with activated charcoal. The obtained perindopril (acid) is isolated in pure form, recrystallized if necessary, then directly reacted with N,N'-dibenzylethylenediamine and converted into benzathine salt.

As the starting substance for the preparation of benzathine salts of perindopril there is used perindopril in the form of a free acid, a hydrate or in the form of a salt with a volatile alkaline component such as the already mentioned perindopril erbumine.

Crystallization is very selective so that also a starting substance of inferior quality can be used, e. g. also with 10 % of diketopiperazine impurity, and nevertheless a product of high purity is obtained. Namely, diketopiperazine impurity does not have a free carboxylic group, therefore it does not crystallize with benzathine and is removed with crystallization mother liquor.

The crystal structure of the compounds was determined by X-ray powder diffractograms (abbr.: XRD) recorded on X'Pert PRO X-ray difractometer with alpha configuration, CuKα radiation, in a range 3-35° 2Θ. FTIR spectra were recorded by Perkin Elmer 727B IR spectrometer.

Benzathine salt of perindopril (abbr: benzathine perindopril) is obtained by the reaction of perindopril and benzathine in a molar ratio 2 : 1 and crystallization from hot ethyl acetate or from water, in a polymorph form A with a characteristic X-ray powder diffractogram represented in Table 1. Therein only signals higher than 15 % of relative intensity are stated.

**Table 1**

| No. | Position [2*Θ*/°] | Spacing [*d*/Å] | Rel. intensity [%] |
|---|---|---|---|
| 1 | 5.8949 | 14.98041 | 25.01 |
| 2 | 6.5122 | 13.56185 | 100.00 |
| 3 | 10.7061 | 8.25681 | 24.99 |
| 4 | 12.0797 | 7.32081 | 17.38 |
| 5 | 14.8462 | 5.96227 | 20.12 |
| 6 | 16.0810 | 5.50712 | 18.65 |
| 7 | 16.6625 | 5.31622 | 24.27 |
| 8 | 17.6098 | 5.03231 | 17.22 |
| 9 | 17.8417 | 4.96743 | 45.19 |
| 10 | 19.0928 | 4.64466 | 47.09 |
| 11 | 15.5830 | 4.52949 | 32.66 |
| 12 | 20.5267 | 4.32333 | 18.58 |
| 13 | 21.3166 | 4.16487 | 31.09 |
| 14 | 21.4948 | 4.13075 | 20.08 |
| 15 | 22.0069 | 4.03576 | 28.04 |
| 16 | 22.6724 | 3.91880 | 15.78 |
| 17 | 23.9340 | 3.71500 | 43.77 |
| 18 | 25.0831 | 3.54736 | 18.66 |
| 19 | 25.5437 | 3.48442 | 16.50 |
| 20 | 28.1560 | 3.16680 | 18.30 |
| 21 | 29.6824 | 3.00733 | 28.99 |
| 22 | 35.0811 | 2.55590 | 20.18 |

Benzathine perindopril in a polymorph form B is obtained by the reaction of perindopril and benzathine in a molar ratio 2 : 1 and crystallization from ethanol subsequent to the addition of methyl tert-butyl ether. It has a characteristic X-ray powder diffractogram, represented in Table 2, wherein only signals with relative intensity higher than 10 % are stated.

**Table 2**

| No. | Position [2*Θ*/°] | Spacing [*d*/Å] | Rel. intensity [%] |
|---|---|---|---|
| 1 | 6.5142 | 13.55760 | 100.00 |
| 2 | 11.5531 | 7.65333 | 28.99 |
| 3 | 12.1045 | 7.30591 | 15.95 |
| 4 | 14.8847 | 5.94695 | 14.10 |
| 5 | 16.0939 | 5.50275 | 13.61 |
| 6 | 17.6270 | 5.02743 | 13.85 |
| 7 | 19.6125 | 4.52273 | 47.32 |
| 8 | 20.1330 | 4.40696 | 14.26 |
| 9 | 20.5802 | 4.31221 | 23.59 |
| 10 | 20.4838 | 4.13283 | 10.67 |
| 11 | 22.0327 | 4.03110 | 22.73 |
| 12 | 23.1710 | 3.83558 | 21.40 |
| 13 | 24.0011 | 3.70477 | 26.78 |
| 14 | 26.1996 | 3.39866 | 11.24 |
| 15 | 26.6917 | 3.00640 | 14.75 |
| 16 | 30.5506 | 2.92381 | 12.96 |
| 17 | 36.6578 | 2.44950 | 13.62 |

New salts of perindopril that are an object of the present invention can be used as therapeutical active substances, which, together with an inert pharmaceutically acceptable carrier, can be transformed into a suitable form such as tablets, which are then used for treatment of cardiovascular diseases, hypertension and heart muscle weakness.

An object of the present invention is also a pharmaceutical formulation containing new benzathine salts of perindopril. As pharmaceutically acceptable adjuvants, substances generally known to those skilled in the art are used.

Inert ingredients or pharmaceutical adjuvants are selected from the following groups:
- fillers such as anhydrous lactose, microcrystalline cellulose, starch, calcium phosphate, calcium carbonate, different sugars and the others;
- binders such as microcrystalline cellulose, hydroxyalkyl celluloses, povidone, cellulose esters, starch or a mixture thereof;
- disintegrants such as starch, cross-linked croscarmellose sodium, crospovidone, microcrystalline cellulose, sodium carboxymethyl cellulose and the others, mostly in an amount of 1-10 %;
- substances for lubrication and improvement of gliding at tabletting such as talc, magnesium stearate, stearic acid, potassium stearate and colloidal silica. These substances are usually added to the other ingredients in final phase.

Pharmaceutical formulations are prepared according to known processes such as direct mixing, dry granulation, wet granulation and tabletting or spraying of a solution of the benzathine salt of perindopril onto inert pharmaceutical substances under simultaneous drying in counterflow of warm air. To the granulate obtained, if necessary, also other ingredients are added, it is homogenized and tabletted in a usual manner.

The benzathine component in the discussed new salts is the cause of a greater hydrophobicity of active substances in this form. Therefore it is realistic to expect a better passage through biological membranes, which also makes possible the manufacture of preparations acting through the skin like creams, ointments or patches.

The transdermal system comprises a back layer unpermeable for the active substance, a polymer layer that serves as a reservoir for the active substance and is pressure-sensitive and a protection foil that is perforated in several sites so that the active substance can permeate through it.

According to the invention, also other single active substances can be added, which improve the basic action of perindopril or act sinergistically. These substances are from the following groups:
- diuretics such as indapamide or hydrochlorothiazide and salts thereof;
- substances with antitrombotic action such as clopidogrel and acetylsalicylic acid;
- substances with anti-hyperlipoproteinemic action such as rosuvastatin;
- inhibitors of calcium ion influx from the group of dihydropiridines such as amlodipine and lacidipine and salts thereof;
- substances with antioxidative action such as dry green tea extract, coenzyme Q₁₀, idebenone, curcumin and others.

Of special interest is the combination with coenzyme Q₁₀ or ubiquinone, which is very active antioxidant and free radical scavenger and, in addition, it can be used at heart diseases associated with a decreased blood flow and too high blood pressure and at indications of heart failure. Recently, also curcumin has turned out to be a very important antioxidant with a strong anti-cancer action.

As expected, benzathine salts of perindopril are surprisingly stable in ageing. Experimentally, a higher stability of benzathine perindopril in comparison with some other known salts of perindopril was determined so that these salts were incubated at 50 °C and 65 % relative humidity in vials with a PE lid. The content within time intervals was determined by HPLC method:
Kromasil C18 column, 5 µm, 150 × 4.6 mm;
mobile phase A: 30 % acetonitrile/70 % buffer pH = 2.0;
mobile phase B: 90 % acetonitrile/10 % buffer pH = 2.0;
gradient from 100 % A to 57 % A within 20 min;
buffer composition: 0.92 g sodium heptane sulphonate and 1 mL of triethylamine/1000 mL water, pH 2.0 with HClO₄;
temperature: 20 °C;
flow: 1mL/min;
detection: UV at 210 nm.

**Table 7. The decrease of perindopril salt content at incubation at 50 °C and 65 % relative humidity:**

| Type of salt: | initial state | 30 days | 60 days | 90 days |
|---|---|---|---|---|
| Benzathine perindopril | 99.8 | 99.2 | 98.7 | 98.1 |
| Perindopril arginine | 99.8 | 98.9 | 98.5 | 97.9 |
| Perindopril erbumine | 99.6 | 98.6 | 97.6 | 96.5 |

The present invention is illustrated by the following Examples, which do not limit it in any way.

### EXAMPLE 1. Benzathine perindopril - polymorph form A

Perindopril (1.97 g, 5.35 mmol) was disolved in 7 mL of water at 50-60 °C and thereto benzathine (0.6 g, 2.5 mmol) was added dropwise under stirring. It was stirred and slowly cooled to room temperature, then it was left to stay at +5 °C over night. The crystal broth was filtered, washed with ice-cold water and dried in vacuum at 50 °C. 2.32 g (90.3 %) of a white crystal salt with a melting point at 84-89 °C (Kofler) were obtained. The XRD powder diffractogram is represented in Table 1 and corresponds to the polymorph form A. The elementary analysis (C, H, N) corresponded to a compound with empirical formula (C₁₉H₃₂N₂O₅)₂ · C₁₆H₂₀N₂ = C₅₄H₈₄N₆O₁₀ and with molecular weight 977.3.

### EXAMPLE 2. Benzathine perindopril - polymorph form A

Perindopril erbumine (2.0 g, 4.53 mmol) was dissolved in 20 mL of ethyl acetate, during stirring benzathine (0.6 g, 2,5 mmol) was added thereto and ethyl acetate was evaporated to dryness in a vacuum at 60 °C. The dry substance was dissolved again in 25 mL of ethyl acetate, ethyl acetate was again evaporated and the dry substance was well dried in vacuum (thereby tert-butlyamine was removed). Subsequently, the dry substance was dissolved in 10 mL of ethyl acetate at 70 °C and 5 mL of n-hexane were added. After cooling the desired benzathine perindopril in the form of fine crystals with a melting point of 82-86 °C was separated. There were obtained 1.97 g (88.9 %) of a substance with XRD diffractogram as stated in Table 1 and corresponding to polymorph form A.

### EXAMPLE 3. Benzathine perindopril - amorphous

Perindopril (1.97 g, 5.35 mmol) was dissolved in 30 mL of water and thereto benzathine (0.64 g, 2.675 mmol) was added dropwise under stirring at 35 °C. It was rapidly dried with dry ice and lyophilized. 2.61 g (100 %) of amorphous benzathine perindopril with a melting point of 80-87 °C were obtained.

### EXAMPLE 4. Benzathine perindopril - polymorph form B

Perindopril (1.0 g, 2.71 mmol) was dissolved in 4 mL of ethanol and thereto benzathine (0.33 g, 1.33 mmol) was added. During stirring 7 mL of methyl tert-butyl ether were added stepwise and it was left for the salt to crystallize over night at +5 °C. It was filtered, washed with methyl tert-butyl ether and dried in vacuum. The crystals (0.67 g, 51.5 %) melt at 84-87 °C (Kofler) and have an XRD powder diffractogram as represented in Table 2, which corresponds to polymorph form B.

### EXAMPLE 5. Benzathine perindopril - polymorph form A

In this Example as a starting substance perindopril of inferior quality (content: 91.5 %, impurity B: 1.4 %, impurity F: 3.1 %) was used. Perindopril (32.4 g, 87.9 mmol) was dissolved in 100 mL of ethyl acetate at 70-75 °C and benzathine (10.4 g, 43.3 mmol) was added during stirring. Then stepwise another 100 mL of n-heptane were added to start the crystallization of the salt. During cooling to room temperature, another 50 mL of n-heptane were added and it was cooled to -15 °C for 4 hours. The salt was filtered and washed well with a mixture of ethyl acetate/n-heptane 1 : 3 and additionally with pure n-heptane. It was dried in a vacuum at 50 °C. There were obtained 40.11 g (93.3 %) of crystals with a melting point of 87-91 °C and XRD diffractogram characteristic for polymorh form A.

The obtained salt was very pure: content 99.8 %, impurity B 0 %, impurity F 0.09 %. This benzathine perindopril was used for the production of test tablets.

### EXAMPLE 6. Tablets with benzathine perindopril - variant I

| Ingredients: | mg/tablet: |
|---|---|
| Benzathine perindopril | 4.43 |
| Lactose DCL 21 | 59.70 |
| Celullose Avicel 102 | 19.87 |
| Corn starch | 5.00 |
| Magnesium stearate | 1.00 |
| Tablet weight | 90.00 mg |

The tablets were prepared by dry mixing and direct tabletting.

### EXAMPLE 7. Tablets with benzathine perindopril - variant II

| Ingredients : | mg/tablet: |
|---|---|
| Benzathine perindopril | 4.43 |
| Lactose DCL21 | 59.70 |
| Celullose Avicel 102 | 18.87 |
| Corn starch | 4.00 |
| Sodium croscarmellose | 2.00 |
| Magnesium stearate | 1.00 |
| Tablet weight | 90.00 mg |

### EXAMPLE 8. Tablets with benzathine perindopril - variant III

| Ingredients: | mg/tablet: |
|---|---|
| Benzathine perindopril | 4.50 |
| Lactose monohydrate | 42.58 |
| Celullose Avicel 102 | 20.42 |
| Sodium hydrogen carbonate | 6.50 |
| Corn starch | 15.00 |
| Magnesium stearate | 1.00 |
| Tablet weight | 90.00 mg |

### EXAMPLE 9. Tablets with benzathine perindopril and indapamide

| Ingredients: | mg/tablet: |
|---|---|
| Benzathine perindopril | 4.50 |
| Lactose monohydrate | 42.58 |
| Celullose Avicel | 20.67 |
| Sodium hydrogen carbonate | 5.00 |
| Corn starch | 15.00 |
| Indapamide | 1.25 |
| Magnesium stearate | 1.00 |
| Tablet weight | 90.00 mg |

### EXAMPLE 10. Tablets with benzathine perindopril and amlodipine besylate

| Ingredients: | mg/tablet: |
|---|---|
| Benzathine perindopril | 4.50 |
| Amlodipine besylate | 5.00 |
| Lactose monohydrate | 42.58 |
| Celullose Avicel | 20.67 |
| Corn starch | 15.00 |
| Silica | 1.25 |
| Magnesium stearate | 1.00 |
| Tablet weight | 90.00 mg |

### EXAMPLE 11. Formulation with benzathine perindopril and diuretic

| Ingredients: | mg/capsule: |
|---|---|
| Benzathine perindopril | 4.43 |
| Hydrochlorothiazide | 12.5 |

### EXAMPLE 12. Formulation with benzathine perindopril and lacidipine

| Ingredients: | mg/tablet: |
|---|---|
| Benzathine perindopril | 8.86 |
| Lactose monohydrate | 66.42 |
| Corn starch | 6.50 |
| Lacidipine | 4.00 |
| Colloidal silica | 0.22 |
| Crospovidone | 3.00 |
| Magnesium stearate | 1.00 |
| Tablet weight | 90.00 mg |

### EXAMPLE 13. Benzathine perindopril with antioxidant

| Ingredients: | mg/tablet: |
|---|---|
| Benzathine perindopril | 8.86 |
| Lactose DCL21 | 53.77 |
| Microcrystalline celullose | 35.57 |
| Curcumin | 35.00 |
| Crospovidone | 4.00 |
| Talc | 1.80 |
| Magnesium stearate | 1.00 |
| Tablet weight | 140.00 mg |

### EXAMPLE 14. Preparation of patch for transdermal application

In a solvent consisting of monomethyl ester of glutaric acid (5 g), ethanol (5 g), butanone (5 g) and 1-dodecanole (7 g), benzathine perindopril ( 4.8 g) was dissolved. Separately, 66 g of a cross-linked acrylate polymer consisting of 2-ethylhexylacrylate, vinyl acetate and acrylic acid in a solvent mixture ethyl acetate/n-hexane/isopropanol/acetyl acetone/toluene = 40/25/25/1/4 (parts by volume) was prepared and during vigorous stirring this solution was added to the previously prepared solution of benzathine perindopril. This was stirred well, another 0.7 g of aluminium acetylacetonate were added and it was stirred at 20 °C for 4 hours to achieve the polymerisation of mixture. Then the resinous sticky substance was spread in a thickness of 0.35 mm on siliconized polyethylene foil and the solvent was removed by drying at 55 °C. The adhesive film was then covered with a thin polyester foil of a thickness of 0.15 mm, which was perforated by a special tool at defined sites.

### EXAMPLE 15. Industrial preparation of benzathine perindopril

a) Perindopril (in acid form) was isolated from the solution after catalytic debenzylation by removing the solvent by distillation until an oily product formed, then heptane was added thereto and the obtained mixture was stirred to obtain a free solid, which was filtered and dried.
b) Purification of perindopril: the crude residue - perindopril in acid form - (172 g) was dissolved in ethyl acetate (516 mL), heated to 35 °C to obtain a clear solution, which was filtered in order to remove insoluble particles. Heptane (2580 mL) was slowly added to this filtrate, which was stirred for 3 hours at 25-30 °C. The solid was filtered, washed with heptane and dried to a constant weight in vacuum. 137 g (79.65 %) of very pure perindopril were obtained.
c) Crystallization of benzathine perindopril: benzathine (34.08 g) was charged in absolute ethanol (143 mL), perindopril (100 g) was slowly added while stirring to obtain a clear solution. This solution was heated at 65-70 °C for 30 minutes and then methyl tert-butyl ether (1842 mL) was slowly added. The solution was cooled to 25-30 °C, stirred for 4 hours, cooled down to 10 °C and then benzathine perindopril (1 g) was added (to initiate the process of crystallization) and the solution was then cooled to -15 °C for 8 hours. The solid was filtered off and washed with cooled methyl tert-butyl ether. After drying of the solid at 50 °C to a constant weight benzathine perindopril (94 g) was obtained.

After concentration of collected filtrates in vacuum, an addition of methyl tert-butyl ether and crystallization, a second yield (16 g) of benzathine perindopril was obtained. The purity of the final product exceeded 99.7 % (by HPLC).

## Claims

1. Salts of perindopril with N,N'-dibenzylethylenediamine (benzathine) of formula: 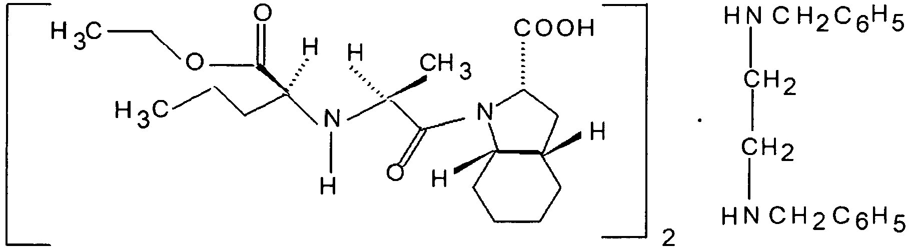 in an amorphous or a crystal form.

2. Benzathine salt of perindopril according to claim 1 in crystal polymorph form A with the following X-ray powder diffractogram:
| No. | Position [2*Θ*/°] | Spacing [*d*/Å] | Rel. intensity [%] |
|---|---|---|---|
| 1 | 5.8949 | 14.98041 | 25.01 |
| 2 | 6.5122 | 13.56185 | 100.00 |
| 3 | 10.7061 | 8.25681 | 24.99 |
| 4 | 12.0797 | 7.32081 | 17.38 |
| 5 | 14.8462 | 5.96227 | 20.12 |
| 6 | 16.0810 | 5.50712 | 18.65 |
| 7 | 16.6625 | 5.31622 | 24.27 |
| 8 | 17.6098 | 5.03231 | 17.22 |
| 9 | 17.8417 | 4.96743 | 45.19 |
| 10 | 19.0928 | 4.64466 | 47.09 |
| 11 | 15.5830 | 4.52949 | 32.66 |
| 12 | 20.5267 | 4.32333 | 18.58 |
| 13 | 21.3166 | 4.16487 | 31.09 |
| 14 | 21.4948 | 4.13075 | 20.08 |
| 15 | 22.0069 | 4.03576 | 28.04 |
| 16 | 22.6724 | 3.91880 | 15.78 |
| 17 | 23.9340 | 3.71500 | 43.77 |
| 18 | 25.0831 | 3.54736 | 18.66 |
| 19 | 25.5437 | 3.48442 | 16.50 |
| 20 | 28.1560 | 3.16680 | 18.30 |
| 21 | 29.6824 | 3.00733 | 28.99 |
| 22 | 35.0811 | 2.55590 | 20.18 |

3. Benzathine salt of perindopril according to claim 1 in crystal polymorph form B with the following X-ray powder diffractogram:
| No. | Position [2*Θ*/°] | Spacing [*d*/Å] | Rel. intensity [%] |
|---|---|---|---|
| 1 | 6.5142 | 13.55760 | 100.00 |
| 2 | 11.5531 | 7.65333 | 28.99 |
| 3 | 12.1045 | 7.30591 | 15.95 |
| 4 | 14.8847 | 5.94695 | 14.10 |
| 5 | 16.0939 | 5.50275 | 13.61 |
| 6 | 17.6270 | 5.02743 | 13.85 |
| 7 | 19.6125 | 4.52273 | 47.32 |
| 8 | 20.1330 | 4.40696 | 14.26 |
| 9 | 20.5802 | 4.31221 | 23.59 |
| 10 | 20.4838 | 4.13283 | 10.67 |
| 11 | 22.0327 | 4.03110 | 22.73 |
| 12 | 23.1710 | 3.83558 | 21.40 |
| 13 | 24.0011 | 3.70477 | 26.78 |
| 14 | 26.1996 | 3.39866 | 11.24 |
| 15 | 26.6917 | 3.00640 | 14.75 |
| 16 | 30.5506 | 2.92381 | 12.96 |
| 17 | 36.6578 | 2.44950 | 13.62 |

4. Benzathine salt of perindopril according to claim 1 in an amorphous form.

5. A process for the preparation of benzathine salts of perindopril of formula: 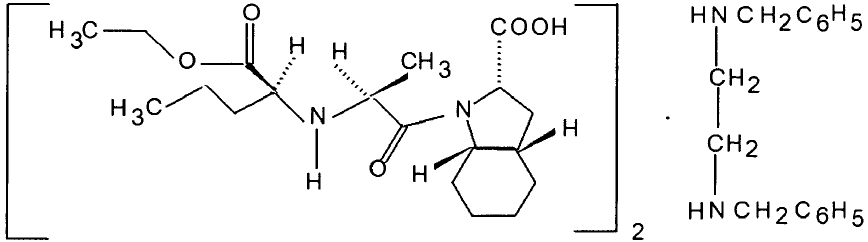 by the reaction of two moles of perindopril with one mole of N,N'-dibenzylethylenediamine.

6. A process for the preparation of benzathine salts of perindopril in crystal form according to claim 5, **characterized in that** at crystallization water or an organic solvent from the group of esters, lower alcohols, ketones, nitriles or ethers, preferably ethyl acetate, acetone, ethanol, acetonitrile and methyl tert-butyl ether is used as a solvent.

7. A process for the preparation of benzathine salts of perindopril in an amorphous form according to claim 5, **characterized in that** the solution of benzathine salt in water is prepared, which is frozen and lyophilized or transformed to a dry state in another manner so that no crystallization occurs.

8. A process for the preparation of a granulate containing benzathine salts of perindopril according to claims 1 to 4, **characterized in that** an aqueous or alcohol/ aqueous solution of benzathine salt of perindopril is sprayed onto a mixture of inert ingredients for the preparation of a granulate and simultaneously dried in a counterflow of warm air with a temperature of not more than 60 °C.

9. A pharmaceutical formulation containing benzathine salts of perindopril according to claims 1 to 4.

10. A pharmaceutical formulation containing benzathine salts of perindopril according to claims 1 to 4, **characterized in that** it additionally contains one or more compounds from the group of diuretics, antitrombolitics, calcium ion influx inhibitors, anti-hyperlipoproteinemics or antioxidants.

11. Pharmaceutical formulation according to claims 9 and 10 for use in the treatment of cardiovascular diseases associated with hypertension, ischemia and heart muscle weakness.

## Patentansprüche

1. Salze von Perindopril mit N,N'-Dibenzylethylendiamin (Benzathin) der Formel: 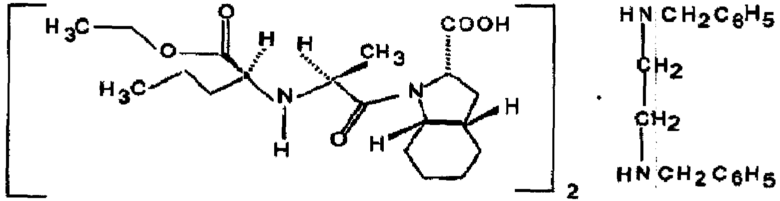 in einer amorphen Form oder einer Kristallform.

2. Benzathinsalz von Perindopril nach Anspruch 1 in Kristallpolymorphform A mit dem folgenden Röntgenpulverbeugungsmuster:
| Nr. | Position [2*Θ*/°] | Abstand [*d*/Å] | Rel. Intensität [%] |
|---|---|---|---|
| 1 | 5,8949 | 14,98041 | 25,01 |
| 2 | 6,5122 | 13,56185 | 100,00 |
| 3 | 10,7061 | 8,25681 | 24,99 |
| 4 | 12,0797 | 7,32081 | 17,38 |
| 5 | 14,8462 | 5,96227 | 20,12 |
| 6 | 16,0810 | 5,50712 | 18,65 |
| 7 | 16,6625 | 5,31622 | 24,27 |
| 8 | 17,6098 | 5,03231 | 17,22 |
| 9 | 17,8417 | 4,96743 | 45,19 |
| 10 | 19,0928 | 4,64466 | 47,09 |
| 11 | 15,5830 | 4,52949 | 32,66 |
| 12 | 20,5267 | 4,32333 | 13,58 |
| 13 | 21,3166 | 4,16487 | 31,09 |
| 14 | 21,4948 | 4,13075 | 20,08 |
| 15 | 22,0069 | 4,03576 | 28,04 |
| 16 | 22,6724 | 3,91880 | 15,78 |
| 17 | 23,9340 | 3,71500 | 43,77 |
| 18 | 25,0831 | 3,54736 | 18,66 |
| 19 | 25,5437 | 3,48442 | 16,50 |
| 20 | 28,1560 | 3,16680 | 18,30 |
| 21 | 29,6824 | 3,00733 | 28,99 |
| 22 | 35,0811 | 2,55590 | 20,18 |

3. Benzathinsalz von Perindopril nach Anspruch 1 in Kristallpolymorphform B mit dem folgenden Röntgenpulverbeugungsmuster:
| Nr. | Position [2*Θ*/°] | Abstand [*d*/Å] | Rel. Intensität [%] |
|---|---|---|---|
| 1 | 6,5142 | 13,55760 | 100,00 |
| 2 | 11,5531 | 7,65333 | 28,99 |
| 3 | 12,1045 | 7,30591 | 15,95 |
| 4 | 14,8847 | 5,94695 | 14,10 |
| 5 | 16,0939 | 5,50275 | 13,61 |
| 6 | 17,6270 | 5,02743 | 13,85 |
| 7 | 19,6125 | 4,52273 | 47,32 |
| 8 | 20,1330 | 4,40696 | 14,26 |
| 9 | 20,5802 | 4,31221 | 23,59 |
| 10 | 20,4838 | 4,13283 | 10,67 |
| 11 | 22,0327 | 4,03110 | 22,73 |
| 12 | 23,1710 | 3,83558 | 21,40 |
| 13 | 24,0011 | 3,70477 | 26,78 |
| 14 | 26,1996 | 3,39866 | 11,24 |
| 15 | 26,6917 | 3,00640 | 14,75 |
| 16 | 30,5506 | 2,92381 | 12,96 |
| 17 | 36,6578 | 2,44950 | 13,62 |

4. Benzathinsalz von Perindopril nach Anspruch 1 in einer amorphen Form.

5. Verfahren zur Herstellung von Benzathinsalzen von Perindopril der Formel: 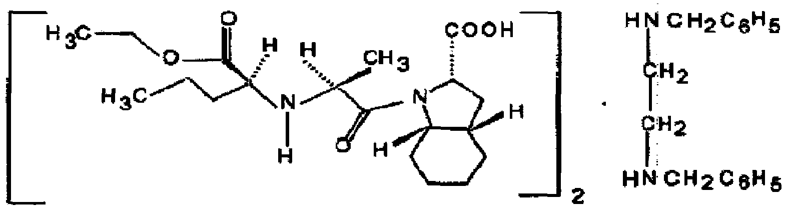 durch die Umsetzung von zwei Molen Perindopril mit einem Mol N,N'-Dibenzylethylendiamin.

6. Verfahren zur Herstellung von Benzathinsalzen von Perindopril in Kristallform nach Anspruch 5, **dadurch gekennzeichnet, dass** man bei der Kristallisation Wasser oder ein organisches Lösungsmittel aus der Gruppe der Ester, niederen Alkohole, Ketone, Nitrile oder Ether, vorzugsweise Essigsäureethylester, von Aceton, Ethanol, Acetonitril und Methyl-tert.-butylether als Lösungsmittel verwendet.

7. Verfahren zur Herstellung von Benzathinsalzen von Perindopril in einer amorphen Form nach Anspruch 5, **dadurch gekennzeichnet, dass** man die Lösung des Benzathinsalzes in Wasser herstellt und diese dann einfriert und lyophilisiert oder auf eine andere Weise in einen trockenen Zustand überführt, so class keine Kristallisation stattfindet.

8. Verfahren zur Herstellung eines Benzathinsalze von Perindopril nach den Ansprüchen 1 bis 4 enthaltenden Granulats, **dadurch gekennzeichnet, dass** man eine wässrige oder alkoholisch/wässrige Lösung des Benzathinsalzes von Perindopril zur Herstellung eines Granulats auf eine Mischung inerter Bestandteile sprüht und gleichzeitig in einem Gegenstrom warmer Luft mit einer Temperatur von nicht mehr als 60°C trocknet.

9. Pharmazeutische Formulierung, welche Benzathinsalze von. Perindopril nach den Ansprüchen 1 bis 4 enthält.

10. Pharmazeutische Formulierung, welche Benzathinsalze von Perindopril nach den Ansprüchen 1 bis 4 enthält, **dadurch gekennzeichnet, dass** sie zusätzlich eine oder mehrere Verbindungen aus der Gruppe der Diuretika, antithrombotischen Mittel, Calciumioneneinstrominhibitoren, antihyperlipoproteinämischen Mittel der Antioxidationsmittel enthält.

11. Pharmazeutische Formulierung nach Anspruch 9 oder 10 zur Verwendung bei der Behandlung von Herz-Kreislauf-Krankheiten, die mit Hypertonie, Ischämie und Herzmuskelschwäche assoziiert sind.

## Revendications

1. Sels de perindopril avec la N,N'-dibenzyléthylènediamine (benzathine) de formule : 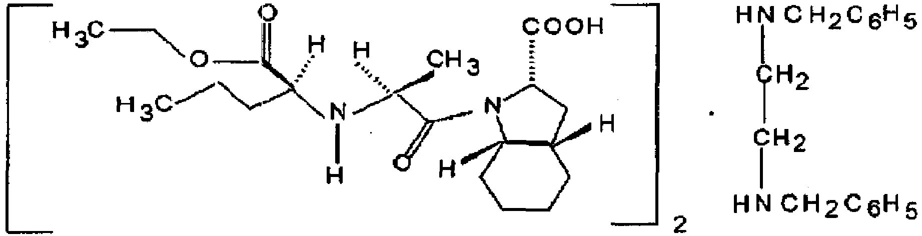 sous forme amorphe ou cristalline.

2. Sel de benzathine du périndopril selon la revendication 1 sous forme polymorphique cristalline A avec le diffractogramme de rayons X de poudre :
| N° | Position [2Θ/°] | Distance [d/Å] | Intensité rel. [%] |
|---|---|---|---|
| 1 | 5,8949 | 14,98041 | 25,01 |
| 2 | 6,5122 | 13,56185 | 100,00 |
| 3 | 10,7061 | 8,25681 | 24,99 |
| 4 | 12,0797 | 7,32081 | 17,38 |
| 5 | 14,8462 | 5,96227 | 20,12 |
| 6 | 16,0810 | 5,50712 | 18,65 |
| 7 | 16,6625 | 5,31622 | 24,27 |
| 8 | 17,6098 | 5,03231 | 17,22 |
| 9 | 17,8417 | 4,96743 | 45,19 |
| 10 | 19,0928 | 4,64466 | 47,09 |
| 11 | 15,5830 | 4,52949 | 32,66 |
| 12 | 20,5267 | 4,32333 | 18,58 |
| 13 | 21,3166 | 4,16487 | 31,09 |
| 14 | 21,4948 | 4,13075 | 20,08 |
| 15 | 22,0069 | 4,03576 | 28,04 |
| 16 | 22,6724 | 3,91880 | 15,78 |
| 17 | 23,9340 | 3,71500 | 43,77 |
| 18 | 25,0831 | 3,54736 | 18,66 |
| 19 | 25,5437 | 3,48442 | 16,50 |
| 20 | 28,1560 | 3,16680 | 18,30 |
| 21 | 29,6824 | 3,00733 | 28,99 |
| 22 | 35,0811 | 2,55590 | 20,18 |

3. Sel de benzathine du périndopril selon la revendication 1 sous forme polymorphique cristalline B avec le diffractogramme de rayons X de poudre :
| N° | Position [2Θ/°] | Distance [d/Å] | Intensité rel. [%] |
|---|---|---|---|
| 1 | 6,5142 | 13,55760 | 100,00 |
| 2 | 11,5531 | 7,65333 | 28,99 |
| 3 | 12,1045 | 7,30591 | 15,95 |
| 4 | 14,8847 | 5,94695 | 14,10 |
| 5 | 16,0939 | 5,50275 | 13,61 |
| 6 | 17,6270 | 5,02743 | 13,85 |
| 7 | 19,6125 | 4,52273 | 47,32 |
| 8 | 20,1330 | 4,40696 | 14,26 |
| 9 | 20,5802 | 4,31221 | 23,59 |
| 10 | 20,4838 | 4,13283 | 10,67 |
| 11 | 22,0327 | 4,03110 | 22,73 |
| 12 | 23,1710 | 3,83558 | 21,40 |
| 13 | 24,0011 | 3,70477 | 26,78 |
| 14 | 26,1996 | 3,39866 | 11,24 |
| 15 | 26,6917 | 3,00640 | 14,75 |
| 16 | 30,5506 | 2,92381 | 12,96 |
| 17 | 36,6578 | 2,44950 | 13,62 |

4. Sel de benzathine du périndopril selon la revendication 1 sous forme amorphe.

5. Procédé de préparation de sels de benzathine de périndopril de formule : 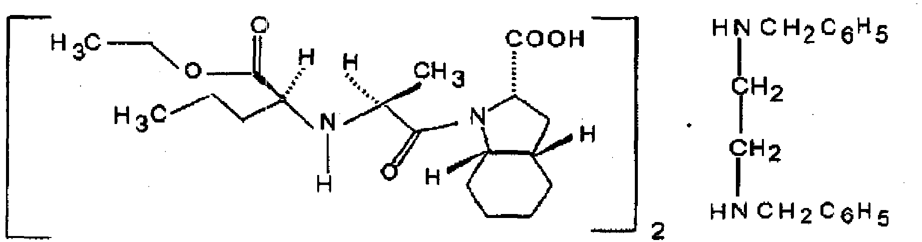 par la réaction de deux moles de périndopril avec une mole de N,N'-dibenzyléthylènediamine.

6. Procédé de préparation de sel de benzathine du périndopril sous forme cristalline selon la revendication 5, **caractérisé en ce que** pour la cristallisation, de l'eau ou un solvant organique choisi dans le groupe constitué par les esters, les alcools courts, les cétones, les nitriles ou les éthers, préférentiellement acétate d'éthyle, l'acétone, l'éthanol, l'acétonitrile et l'éther de méthyle et de tert-butyle est utilisé comme solvant.

7. Procédé de préparation de sels de benzathine de périndopril sous une forme amorphe selon la revendication 5, **caractérisé en ce que** la solution de sel de benzathine dans l'eau est préparée, pour être congelée et lyophilisée ou transformée vers un état sec de toute autre façon de sorte à ce qu'aucune cristallisation ne se produise.

8. Procédé de préparation d'un granulat contenant des sels de benzathine de périndopril selon les revendications 1 à 4, **caractérisé en ce qu'**une solution aqueuse ou alcoolique/aqueuse de sel de benzathine de périndopril est vaporisée sur un mélange de composants internes pour la préparation d'un granulat et simultanément séchée à l'air chaud à contre-courant à une température ne dépassant pas 60 °C.

9. Formule pharmaceutique contenant des sels de benzathine de périndopril selon les revendications 1 à 4.

10. Formule pharmaceutique contenant les sels de benzathine de perindopril selon les revendications 1 à 4, **caractérisée en ce qu'**elle contient en outre un ou plusieurs composés du groupe des diurétiques, antithrombotiques, inhibiteurs de l'influx des ions calcium, antihyperlipoprotéinémiques ou antioxydants.

11. Formule pharmaceutique selon les revendications 9 et 10 pour utilisation dans le traitement des maladies cardiovasculaires associées à l'hypertension, l'ischémie et la faiblesse du muscle cardiaque.
